(19) Europäisches Patentamt
European Patent Office
Office européen des brevets

(11) Numéro de publication: 0 247 954
A1

(12) **DEMANDE DE BREVET EUROPEEN**

(21) Numéro de dépôt: 87420139.5

(22) Date de dépôt: 20.05.87

(51) Int. Cl.4: C 07 D 233/92

(30) Priorité: 29.05.86 FR 8607917

(43) Date de publication de la demande:
02.12.87 Bulletin 87/49

(84) Etats contractants désignés:
AT BE CH DE ES FR GB GR IT LI LU NL SE

(71) Demandeur: RHONE-POULENC CHIMIE
25, quai Paul Doumer
F-92408 Courbevoie Cédex (FR)

(72) Inventeur: Desbois, Michel
526 Chemin du bois
F-69140 Rilleux La Pape (FR)

(74) Mandataire: Cazes, Jean-Marie et al
RHONE-POULENC INTERSERVICES Service Brevets
Chimie 25, quai Paul Doumer
F-92408 Courbevoie Cédex (FR)

(54) **Procédé de préparation du méthylnitroimidazole.**

(57) La présente invention concerne un nouveau procédé de préparation de méthylnitroimidazole par mise en contact au sein de l'acide flurohydrique de méthylimidazole et d'un agent nitrant.

EP 0 247 954 A1

**Description**

PROCEDE DE PREPARATION DE METHYLNITROIMIDAZOLE

La présente invention concerne un procédé de préparation de méthylnitroimidazole. Elle concerne plus particulièrement un procédé de nitration du méthylimidazole.

Il est connu dans l'art antérieur plusieurs procédés de nitration du méthylimidazole mais tous utilisent l'acide sulfurique comme milieu réactionnel.

D'après le brevet américain US 4 209 631 la nitration est effectuée dans l'acide sulfurique à une température comprise entre 110°C et 140°C, les rendements en nitrométhylimidazole obtenus ne dépassent jamais 80 %.

Il est aussi connu d'après le brevet allemand nº 2 645 172 un procédé de nitration effectué aussi dans l'acide sulfurique mais en présence d'urée et à une température d'environ 125°C. Les rendements annoncés en méthylnitroimidazole n'atteignent pas 85 %.

Le dernier procédé connu d'après le brevet roumain numéro 51 138 permet d'atteindre des rendements en méthylnitroimidazole d'environ 95 %. Ce procédé consiste à nitrer le méthylimidazole dans l'oléum contenant 1 à 30 % de $SO_3$.

L'ensemble des procédés précédemment décrits utilise toujours comme milieu réactionnel l'acide sulfurique. L'utilisation de l'acide sulfurique en tant que milieu réactionnel présente cependant quelques inconvénients. D'un point de vue technique, l'acide sulfurique étant un produit présentant une viscosité élevée l'agitation du milieu est difficile, en outre l'acide sulfurique présente un point d'ébullition trop élevé pour être facilement recyclable ce qui entraine des problèmes de pollution lors de l'élimination des effluents de la réaction que sont les sulfates.

D'un point de vue chimique l'acide sulfurique est un milieu oxydant et sulfonant qui outre les dérivés désirés donne naissance à des produits secondaires oxydés ou sulfonés non utilisables.

Il a été decouvert et c'est l'objet de la présente invention un nouveau procédé permettant d'éviter ces inconvénients techniques et chimiques. En effet l'acide sulfurique n'est pas utilisé comme milieu réactionnel contrairement à tous les procédés connus jusqu'à ce jour.

Le procédé de préparation du méthylnitroimidazole objet de la présente invention est caractérisé par le fait que le méthylimidazole est mis en contact avec un agent nitrant au sein de l'acide fluorhydrique liquide.

La réaction chimique de nitration peut être écrite selon le schéma réactionnel suivant :

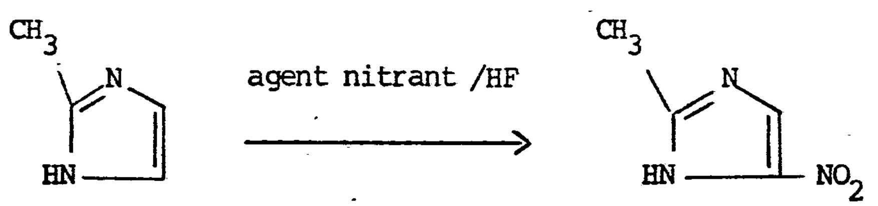


L'agent nitrant peut être choisi de façon non limitative parmi les différents acides nitriques disponibles, les sels alcalins de l'acide nitrique ou les sels de nitronium.

Parmi les acides nitriques disponibles on peut utiliser l'acide nitrique concentré à 65 % en poids ou l'acide nitrique fumant à 93 % minimum en poids. On préfère utiliser l'acide nitrique fumant.

L'acide fluorhydrique utilisé comme milieu réactionnel est de préférence anhydre. Bien que le caractère anhydre ne soit pas un critère essentiel, l'eau faisant diminuer les rendements réactionnels et étant toujours apportée au moins en partie par l'acide nitrique on préfère utiliser l'acide fluorhydrique exempt d'eau.

Le rapport molaire de l'acide fluorhydrique au méthylimidazole est de préférence compris entre 5 et 50 et encore plus préférentiellement compris entre 10 et 20. Ces conditions de molarité ne sont pas des critères essentiels de mise en oeuvre de la présente invention mais uniquement des préférences pour une meilleure économie du procédé

Le rapport molaire de l'agent nitrant au méthylimidazole est de préférence compris entre 1 et 1,5 et encore plus préférentiellement compris entre 1 et 1,1.

La température réactionnelle peut varier dans de larges limites, elle est néanmoins de préférence comprise entre -20° et 150°C et encore plus avantageusement comprise entre 0 et 20°C.

La pression sera adaptée simplement à la température de la réaction afin de conserver l'acide fluorhydrique sous forme liquide. Ainsi lorsque la réaction a lieu à une température supérieure à 20°C, c'est à dire au dessus du point d'ébullition de l'acide fluorhydrique, la pression sera de préférence supérieure à la pression atmosphérique.

L'acide fluorhydrique solvant de la réaction est récupéré en fin de réaction par distillation ou par tout autre méthode connue de l'homme de l'art.

Le méthylnitroimidazole après élimination de l'acide fluorhydrique se trouve sous forme de fluorhydrate d'amine. Il est obtenu sous forme non salifiée soit par chauffage en présence d'un solvant tel que par exemple le flugène ®113 ou le tétrachlorure de carbone soit par déplacement chimique à l'aide d'un composé basique puis extraction de l'amine par tout solvant connu de l'homme de l'art.

Les composés obtenus selon la présente invention sont utilisés comme intermédiaires de synthèse dans

l'industrie pharmaceutique.

La présente invention sera plus complètement décrite à l'aide des exemples suivants qui ne doivent pas être considérés comme limitatifs de l'invention.

EXEMPLE 1

Nitration du méthylimidazole

Dans un réacteur inox de 250 ml agité par barreau aimanté, on introduit successivement : 100 g (5 moles) d'acide fluorhydrique anhydre, 16,5 g (0,2 mole) de méthylimidazole (dissolution exothermique) et 14,5 g (0,22 mole) d'acide nitrique 98 %. Le réacteur est fermé et laissé sous agitation à environ 0° C pendant 10 minutes. Après réaction, le mélange brut acide est coulé sur 200 g de glace pilée puis neutralisé jusqu'à pH environ 2 par une solution de KOH à 40 %. Le précipité obtenu est filtré et séché. Les analyses par chromatographie liquide haute performance et infrarouge et IR effectuées sur les phases aqueuses acides et sur le précipité nous montrent un taux de transformation et un rendement d'environ 100 %

EXEMPLE 2

Récupération de l'acide fluorhydrique en présence de $H_2SO_4$ concentré

La réaction de nitration est réalisée de manière identique à l'exemple 1 avec toutefois 50 g (2,5 moles) d'acide fluorhydrique au lieu de 100 g. En fin de réaction, on introduit 25 g (0,25 m) d'$H_2SO_4$ concentré dans le mélange brut réactionnel lequel est porté de 30 à 80° C pendant 7 heures (le mélange est resté 3h 30 à 80° C). L'acide fluorhydrique évaporé est piégé dans l'eau glacée. Après traitement et analyses, le mononitroimidazole obtenu se présente sous forme d'une pâte marron foncée. Le bilan sur l'acide fluorhydrique nous montre que le taux de récupération de l'HF est de 84 %.

EXEMPLE 3

Récupération de l'acide fluorhydrique en présence d'oléum 63 %

On procède de manière identique à l'essai 2 en présence de 30 g d'oléum 63 %. La distillation de l'HF s'effectue de 20 à 80° C en 2h 40. Après traitement et analyses, le mononitroimidazole obtenu se présente sous forme d'une pâte noire. Le taux d'évaporation de l'HF est 96,4 %.

EXEMPLE 4

Récupération de l'acide fluorhydrique par entraînement au flugère 113 ($CF_2Cl - CFCl_2$)

La réaction de nitration est réalisée de manière identique à l'essai 1. On plonge ensuite le réacteur contenant le mélange brut réactionnel dans un bain d'huile à 60° C, tout en additionnant en contenu, pendant 2 heures, 240 cm3 de flugène R 113 que l'on condense en sortie de réacteur dans un récipient refroidi par un bain d'eau glacée en contenant 190 g d'eau. La température du milieu réactionnel s'élève progressivement jusqu'à 38° C pendant cette opération. Après traitement et analyses, le mononitroimidazole obtenu se présente sous forme d'une poudre beige claire. Le taux d'évaporation de l'HF est de 60 %.

EXEMPLE 5

Récupération de l'acide fluorhydrique par entraînement au tétrachlorure de carbone ($CCl_4$)

La réaction de nitration est réalisée de manière identique à l'essai 1. On porte ensuite le réacteur contenant le mélange brut réactionnel dans un bain d'huile à 80° C, tout en additionnant en continu, pendant 2 h 15 mn, 268 cm3 de $CCl_4$ que l'on condense en sortie de réacteur dans un récipient refroidi par un bain acétone-carboglace. La température du milieu réactionnel s'élève progressivement jusqu'à 56° C. Il est remarquer que :

- le distillat se présente sous forme d'un système biphasique parfaitement incolore [$CCl_4$ - HF ($H_2O$)] ;
- le mélange réactionnel contenant $CCl_4$, mononitroimidazole et le reste de HF est homogène.

Après traitement et analyses, le mononitroimidazole obtenu se présente sous forme d'une poudre beige claire. Le taux d'évaporation de HF est de 75,4 %.

**Revendications**

1) Procédé de préparation de méthylnitroimidazole caractérisé en ce qu'on met en présence le méthylimidazole et un agent nitrant dans l'acide fluorhydrique.

2) Procédé selon la revendication 1, caractérisé en ce que l'agent nitrant est choisi parmi l'acide nitrique, les sels alcalins de l'acide nitrique, les sels de nitronium.

3) Procédé selon les revendications 1 et 2, caractérisé en ce que l'agent nitrant est l'acide nitrique.

4) Procédé selon la revendication 1, caractérisé en ce que l'acide fluorhydrique est anhydre.

5) Procédé selon la revendication 1, caractérisé en ce que le rapport molaire de l'acide fluorhydrique ou méthylimidazole est compris entre 5 et 50 et de préférence entre 10 et 20.

6) Procédé selon la revendication 1, caractérisé en ce que le rapport molaire de l'agent nitrant au méthylimidazole est compris entre 1 et 1,5 et de préférence entre 1 et 1,1.

7) Procédé selon la revendication 1, caractérisé en ce que la température réactionnelle est comprise entre -20° et 150° C et de préférence entre 0 et 20° C.

Office européen des brevets

# RAPPORT DE RECHERCHE EUROPEENNE

Numero de la demande

EP 87 42 0139

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-3 326 983 (VESELY-LINN)<br>--- | | C 07 D 233/92 |
| A | JOURNAL OF ORGANIC CHEMISTRY, vol. 32, no. 1, janvier 1967, pages 250-251, Easton, Pennsylvania, US; J. LENARD: "Nitration and denitration in hydrogen fluoride"<br>* Page 250, colonne 2, en haut *<br>--- | | |
| A | TETRAHEDRON LETTERS, no. 17, avril 1975, pages 1429-1430, Pergamon Press, Oxford, GB; C. COMNINELLIS et al.: "Reactions of organic compounds in aqueous hydrofluoric acid"<br>--- | | DOMAINES TECHNIQUES RECHERCHES (Int. Cl.4) |
| A | DE-A-2 219 216 (BAYER)<br>--- | | C 07 D 233/00 |
| A | DE-A-2 438 210 (CIBA-GEIGY)<br>----- | | |

Le présent rapport de recherche a été établi pour toutes les revendications

| Lieu de la recherche | Date d'achèvement de la recherche | Examinateur |
|---|---|---|
| LA HAYE | 07-08-1987 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82